# EUROPEAN PATENT SPECIFICATION

(11) **EP 1 051 468 B1**
(45) Date of publication and mention of the grant of the patent: **16.06.2004**
(21) Application number: 99908817.2
(22) Date of filing: 22.01.1999
(51) Int. Cl.: C11D 1/00

(54) **SKIN CLEANSING BAR COMPOSITION**
HAUTREINIGUNGSSTÜCK
COMPOSITION SOUS FORME DE PAIN POUR LE DEMAQUILLAGE DE LA PEAU

(30) Priority: 26.01.1998 US 12989; 26.01.1998 US 12990
(43) Date of publication of application: 15.11.2000
(73) Proprietor: UNILEVER PLC, London EC4P 4BQ (GB); UNILEVER N.V., 3013 AL Rotterdam (NL)
(72) Inventor: HE, Mengtao, Scottsdale, Arizona 85259 (US); BARRATT, Michael, 45 River Road, Edgewater, NJ 07020 (US); DALTON, James, Joseph, Scottsdale, AZ 85254 (US); FAIR, Michael, Joseph, 45 River Road,Edgewater, NJ 07020 (US); PETKO, Michael, Francis, 45 River Road, Edgewater, NJ 07020 (US); SHEEHAN, John, Gerard, 800E 17th Street, Brooklyn, NY 11230 (US); KHAN-LODHI, Abid, Nadim, Edgewater, NJ 07020 (US); McFANN, Gregory, Jay, 45 River Road, Edgewater, NJ 07020 (US); FARRELL, Terence, James, 45 River Road, Edgewater, NJ 07020 (US)
(74) Representative: Elliott, Peter William
(86) International application number: PCT/EP1999/000424
(87) International publication number: WO 1999/037744

(56) References cited:
- WO-A-97/49381
- GB-A- 488 196
- GB-A- 1 469 131
- US-A- 5 510 050
- US-A- 5 658 868

## Description

The present invention relates to personal wash bar compositions, particularly compositions comprising (1) one or more surfactants, (2) one or more liquid emollient oils and/or humectants, and (3) a solid polyol ester with specifically defined HLB and melting temperature. The invention relates to structuring high levels of emollient oils and/or humectants in solid bar matrixes using the specific polyol ester solid. Through careful balancing of the polyol ester to oil and/or humectant ratio, and carefully balancing the structuring systems for the oil domain and surfactant domain in the solid bar, a novel approach of delivering mild, moisturizing ingredients to the skin via personal wash is developed.

It is technically difficult to include high levels (e.g., 10-20%) of liquid hydrophobic emollient oil (e.g., sunflower seed oil) and/or hydrophilic liquid humectant (e.g., glycerin) in a solid personal washing bar form, and meanwhile maintain the bar mildness and benefit delivery to the human skin.

For example, high levels of humectants (e.g., glycerin, low MW polyalkylene glycol) can be trapped in a solid matrix of carboxylic fatty acid soap bar. However, it is known that carboxylic soap is harsh to skin, especially when the soap concentration is high, and when the soap is dissolved in the aqueous washing liquor through its own hydrophilic tendency, or through the act of cosurfactants in the bar.

On the other hand, non-soap synthetic bar formulations are primarily structured by either hydrophobic crystalline materials such as free fatty acid or paraffin wax, or by hydrophilic crystalline materials such as polyalkylene glycol of high molecular weight (e.g., MW between 2000 and 20000). While not wishing to be bound by theory, formulating high levels of emollient oil into a bar structured by the hydrophobic crystalline materials is believed to result in tightly binding the oil with the hydrophobic crystalline structurants. This contributes to the incapability of releasing the oil to water from the bar, and may prevent delivering the oil to the skin via personal wash (see Example 1). Including liquid oils and/or liquid humectants in bars structured by hydrophilic crystalline materials leads to another difficulty. That is, the oil and the humectant are not compatible with the hydrophilic structurants, and this incompatibility results in oil leakage and phase separation from the bulk portion of the bar (see Example 1).

Therefore, a novel mild bar structuring system is required to be able to satisfactorily structure the oil and/or the humectant in the bar, while simultaneously permitting oil release from the bar to the aqueous liquid and then to the human skin via the route of personal wash.

Novel to the art, the applicants of the present invention found that a specific group of polyol esters (i.e., having specific ranges of HLB and specific melting temperature) are capable of both structuring a high level of hydrophobic emollient oils and/or humectants in solid form (e.g., bars) while still permitting the oil and humectants to be released from the solid into aqueous liquor, to be delivered to the human skin through the route of personal wash. Using the polyol esters with specific hydrophilic-lipophilic balance (HLB), balancing the ratio between polyol ester and oil/humectant, and balancing the ratio between polyol ester to other structurants are critical to achieve the desired oil structuring and oil releasing.

The use of polyol esters in personal washing bars is not new.

European Patent EP-A-0617955 assigned to Kao Corp. (invented by M. Tonomura and T. Ohtomo), for example, teaches the use of monoglycerides to boost the lather of formulations comprising only nonionic surfactants. The application does not teach the use of the combination of specific solid monoglycerides and high level of liquid emollient oils/humectants to make bars, preferably pourable, cast melt bars, preferably comprising anionic surfactants and amphoteric surfactants. By contrast, the subject invention found that by using a specific polyol ester (e.g., specific range of HLB, polyol ester to oil/humectant ratio, and polyol ester to other structurant ratio), high levels of emollient oil and/or humectants can be satisfactorily incorporated into bars for skin benefit.

WO-4-92/13060 to Procter & Gamble (authored by R. James) teaches the use of monoglycerides in general, PEG and fatty acid as binders for an extruded syndet bar formulation. The prior art, however, does not teach the use of a specific combination of specific monoglycerides (e.g., specific ranges of HLB), PEG and fatty acid to incorporate high level of liquid emollients (e.g., vegetable oils) or liquid humectants (e.g., glycerin) to make a bar, preferably a pourable, cast melt bar. The prior art also does not teach the specific bar formulation spaces to ensure that high levels of liquid oils and/or humectants can be structured in the solid bar matrixes, and can be delivered to skin via personal wash.

In contrast, in the present invention it has been found that by using a specific polyol ester (eg, polyol esters with specific range of HLB, specific polyol ester to oil ratio, and specific polyol ester to other structurant (eg, PEG and fatty acid ratio), high levels of emollient oil and/or humectants can be satisfactorily incorporated into bars, and can be delivered from the bars to skin under the personal wash condition.

US Patent No 5,510,050 to J Dunbar,P Beerse, and E Walker also teaches the use of monoglycerides in general as a non-preferred candidate for the plasticizers in an extruded cleansing bar containing liquid polyols (4-15%) and magnesium soap (4.5 to 50%). The preferred plasticizers are fatty acids, sodium soap, and paraffin wax (Column 5, line 32-24).

International patent application WO 97/49381 discloses a soap chip composition which in use allows deposition of benefit agent without comprising processing. The soap chip composition comprises 40% to about 80% by weight of chip composition alkylene glycol having a molecular weight from 4,000 to 100,000, 10% to 40% by weight chip composition benefit agent, 0.01 to 10% by weight chip composition fumed silica, 0 to 10% by weight chip composition, water, and 0% to 15% by weight chip composition C₉ to C₂₂ fatty acid.

The prior art, however, does not teach the use of a specific combination or specific monoglycerides (eg specific ranges of HLB) and other plasticizers to incorporate high level of liquid emollients (eg, vegetable oils) / liquid humectants (eg, glycerin) into a bar, preferably pourable, cast melt bar. The prior art also does not teach the specific bar formulation spaces to ensure that high levels of liquid oils and/or humectants can be structured in the solid bar matrixes and can be delivered to skin via personal wash. In fact, as found by the subject invention, the preferred plasticizers used in the prior art hinder the skin deposition of liquid oils from bars to skin. The prior art has to use magnesium soap as the key ingredient to aid processing (column 2, line 26).

In contrast, the present invention has found that by using a specific polyol ester (e.g., polyol esters with specific range of HLB, specific polyol ester to oil ratio, and specific polyol ester to other structurant (e.g., PEG and fatty acid) ratio), high levels of emollient oil and/or liquid humectants can be satisfactorily incorporated into bars, and can be delivered from the bars to skin under the personal wash condition. In the present invention, carboxylic acid soap in general is an optional ingredient. This soap may cause skin irritation, and is preferred to be used at about below 4% wt. of total bar composition in the subject application.

GB Parent 1,570,142 assigned to GAF Corp. teaches the use of both hardened triglycerides and fatty alcohols as the plasticizers in an extruded syndet formulation. In contrast to the subject invention, the prior art does not teach the use of the combination of specific monoglycerides and high level of liquid emollient/humectants to make bars. By contrast, the subject invention found that by using a specific polyol ester (e.g., specific range of HLB, polyol ester to oil ratio, and polyol ester to other structurant ratio), high levels of emollient oil and liquid humectants can be satisfactorily incorporated in the bar, and can be delivered to skin via skin cleansing for the purpose of skin moisturization.

In summary, none of the references, alone or in combination teach that the use of specific polyol esters (e.g., having a specific melting temperature, especially specific hydrophobic-lipophobic balance (HLB)) in specific bar compositions (i.e., containing greater than or equal to 5% hydrophobic emollient oils and/or humectants, the polyol ester to oil/humectant ratio being greater or equal to 1:1, and the polyol ester to other structurant ratio being greater than 1:1) will result in bars, preferably pourable cast melt bars, with enhanced oil/humectant carrying and releasing capabilities. These capabilities are crucial to benefit delivery to the skin via personal wash.

In another embodiment, the invention comprises adjuvant compositions comprising (1) one or more surfactant, (2) one or more liquid emollient oils and/or humectants and(3) solid polyol ester with defined HLB and melting temperature. In particular, this embodiment relates to chip compositions comprising emollient chips entrapped and/or dissolved in the specific solid polyol ester as the thickened carrier. The emollient containing thickened carrier compositions are formed as separate chip/powder/granule compositions (referred to as adjuvant chips), and mixed with "base" chips (comprising the surfactant system) prior to milling, extruding and stamping the bars.

U.S. Patent No. 5,154,849 to Visscher et al. teaches bar compositions containing a silicone skin mildness/moisturizing aid component. In one embodiment, the silicone component may be mixed with a carrier which is selected to facilitate incorporation of the silicone. At column 16, the reference describes that silicone is mixed into melted Carbowax (i.e., polyethylene glycol), that the mixture is cooled to form flakes, and that the flakes are preferably added to an amalgamator.

It is clear, however, that the Visscher et al. contemplates a silicone/carrier system different from the adjuvant chips of the subject invention. First, the Visscher patent does not teach selecting a carrier having a specific HLB to both carry high levels of oils, and permit oil release from the solid into water. For example, polyethylene glycol (HLB>18) is not miscible with most of hydrophobic oils such as silicone oil or vegetable oil at mixing temperatures (e.g., 70-120°C), and upon cooling, oil tends to leak out of the PEG solid matrix. Therefore PEG has a poor oil-carrying capacity, although it permits oil release from oil into water, and then to skin via personal wash (see Example 1).

On the other hand, fatty acid, ethers, alcohols or paraffin wax (HLB <2) have high oil carrying capability (Example 1); however, it is difficult to have oils released from those hydrophobic solids into water, and then to skin at conditions relevant to personal wash.

Novel to the art, the subject invention has found that specific solid polyol esters (i.e., specific HLB between 2.5 and 15) are capable of carrying high levels of oil/humectant and simultaneously providing oil release from the solid into water then to skin via personal wash.

U.S. Patent applications filed by Unilever uses additional thickeners such as fumed silica or additional hydrophobically modified polyalkylene glycols or EO-PO copolymers to improve the oil-carrying capability of polyalkylene glycol in the adjuvant chips, and to modify the dissolution speed of the adjuvant chips in water. Nevertheless, those applications use highly hydrophilic materials such as PEG and EO-PO (HLB»15), and do not teach selecting a carrier having specific HLB (between 2.5 and 15, preferably between 2 and 8) to not only to carry high levels of oils, but also to permit oil release from the solid into water.

For example, polyethylene glycol (HLB>18) or hydrophobically modified PEG claimed (HLB>15) are not miscible with most of hydrophobic oils such as silicone oil or vegetable oil at mixing temperatures (e.g., 70-120°C), and, upon cooling, oil tends to leak out of the solid matrix (see Example 1). Thus thickeners such as fumed silica have to be added to improve the adjuvant's oil-carrying capacity. Nevertheless, fumed silica is in the form of very fine powders (i.e., 7-30 millimicrons), which increase the processing difficulties and potentially increase the cost.

By contrast, the present invention teaches the use of specific solid polyol esters (i.e., specific HLB between 2 and 15) to carry high levels of oil/humectant, and provide oil release from the solid into water then to skin via personal wash, without or with a reduced level of thickeners such as fumed silica (e.g., 0-0.5%). This is advantageous because potentially processing can be simplified, and costs can be reduced.

The references, alone or in combination, fail to teach that the use of specific polyol esters (e.g., having specific melting temperature, especially specific hydrophobic-lipophobic balance (HLB)) in specific adjuvant chips, flakes. or granules or powders (i.e., containing greater than or equal to 5% by weight of the chip composition hydrophobic emollient oils and/or humectants in the adjuvant chips, the polyol ester to oil/humectant ratio being greater than or equal to 1:1, and the polyol ester to other structurant ratio greater than 1:1) will result in bars, processed by adjuvant technology, with enhanced oil/humectant carrying and releasing capabilities. These capabilities are crucial to benefit delivery to the skin via personal wash.

Not to be bound by the theory, it is believed that the adjuvant chips of the subject invention entrap emollient oils by a mechanism that is different from those of prior art. That is, hydrophobic oils such as sunflower seed oil tend to be miscible with the polyol esters during mixing (temperature between 65-120°C) to form a one phase isotropic liquid. Upon cooling, the oil may not be in the form of discrete droplets as is found in the adjuvant chips where polyalkylene glycol is the major carrier. Instead, oils may exist in the crystalline cracks or even in the form of solid solution in the chips of the present invention. The chemical affinity of oil and polyol ester is believed to contribute significantly to the stability of the oils in the polyol ester carrier.

In one embodiment, the applicants have found that in personal wash bar compositions comprising (%: percentage in weight):
(a) 5% to 70%, preferably 10% to 60%, of the total composition of surfactant or mixtures of surfactants; and
(b) 5% to 40%, preferably 10% to 25% of the total bar composition of a liquid hydrophobic emollient oil, liquid hydrophilic humectant or a mixture thereof;
   The liquid hydrophobic emollient oil has a solubility of less than 10%, preferably less than 5 %, and most preferably less than 1% in water at 25°C.
   The liquid emollient oil has a melting temperature of less than 25°C and has a viscosity less than 105 centipoise, preferably less than 50,000 centipoise, most preferably less than 10,000 centipoise at 25°C.
   The emollient oil is selected from hydrocarbon oils, silicones; liquid diglycerides, liquid tri- glycerides, liquid di- and tri- glyceride derivatives, vegetable oils, liquid hydrocarbon esters, silicones, sterols, lanolins and sunscreen oils, and mixtures thereof.
   The liquid hydrophilic humectant has a solubility of greater than or equal to 50% wt. in water at 25°C.
   The liquid humectant has a melting temperature at less than 25°C and has a viscosity at less than 5000 centipoise, preferably less than 1000 centipoise.
   The liquid humectant is selected from polyols consisting of glycerol, glycerin, C1-C10 alkylene glycols such as propylene glycol, liquid polyalkylene glycols such as polypropylene glycols, and polyethylene glycols with molecular weight less than 1000 (such that they are in liquid state at 25°C), ethyl hexanediol, and hexylene glycols.
(c) 15% to 70%, preferably 20% to 50% of the total composition of a solid amphiphilic polyol ester with general molecular structure described as 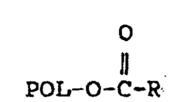 in which POL represents the polyol moiety, R represents an organic hydrophobic group (e.g., straight or branched chain C8-C24 alkyl or alkylene), and one or more R-(C=O)-O- functional groups are chemically attached to one or more of the hydroxy groups of the polyol moiety (POL) to achieve partial or total esterification;
   the solid, amphiphilic polyol ester having a hydrophilic-lipophilic balance (HLB) number at between 2 and 15, preferably between 2.5 and 10, more preferably between 3 and 8;
   the polyol ester having a melting temperature at between 40°C and 90°C, preferably at between 45°C and 70°C;
   the weight ratio of the polyol ester (c) to the sum of the emollient oil and/or humectants (b) being greater than or equal to 1:1, preferably greater than or equal to 1.5:1, most preferably greater than or equal to 2.0:1; .
   the solid, amphiphilic polyol ester include but not being limited to glycerin fatty esters, such as glyceryl monolaurate and glyceryl monostearate; alkylene glycol fatty esters, such as ethylene
   glycol monostearate and ethylene glycol monolaurate; pentaerythrityl fatty esters such as pentaerythrityl stearate; polyglycerin fatty esters such as hexaglyceryl tristearate;
such that high levels of lipophilic oils and/or liquid humectants can be satisfactorily incorporated in the soli matrixes of the bars while retaining the releasing abilit to deliver preferred skin benefits to skin via skin cleansing.

In another embodiment, the invention comprises an adjuvan composition in the forms of chips, powders, granules or mixtures thereof comprising (% by wt.):
(1) 50% to 95%, preferably 65% to 90% of total chip composition a solid, amphiphilic polyol ester having the following structure described as 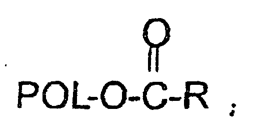
wherein POL represents the polyol moiety, R represents an organic hydrophobic group, and one or more R-(C=O)-O- functional groups are chemically attached to one or more hydroxy groups of the polyol moiety to achieve partial or total esterification;
the solid, amphiphilic polyol ester having a hydrophilic-lipophilic balance (HLB) number at between 2 and 15, preferably between 3 and 8;
the polyol ester having melting temperature at between 40°C and 90°C, preferably at between 45°C and 70°C;
the solid, amphiphilic polyol ester includes but not being limited to glycerin fatty esters, such as glyceryl monolaurate and glyceryl monostearate;
alkylene glycol fatty esters, such as ethylene glycol monostearate and ethylene glycol monolaurate; pentaeryrthrityl fatty esters such as pentaeryrthrityl stearate; polyglycerin fatty
esters such as hexaglyceryl tristearate; and
(b) 5% to 50%, preferably 10% to 35%, most preferably 10% to 25% of the chip composition of a liquid hydrophobic emollient oil, liquid hydrophilic humectant or a mixture thereof;
   the weight ratio of the polyol ester as carrier (1) in (a) to the sum of said emollient oil and/or humectants (b) being greater than or equal to 1:1, preferably greater than or equal to 1.5:1; this carrier to emollient ratio is a criticality because below this ratio, oil and humectant tend to separate from the bulk of the solid matrix.

In yet another embodiment, the invention comprises an extruded bar composition which is produced using about 5 to 80%, preferably 10 to 50%, most preferably 20% to 40% said adjuvant compositions in the form of solid chips, flakes, powders, granules or mixtures thereof; and about 20 to 95% of a surfactant system (base) in the form of chips, flakes, granules or mixtures thereof, wherein the surfactant is selected from anionic surfactants, amphoteric surfactants, nonionic surfactants, cationic surfactants and mixtures thereof. The surfactant system may also contain minor amounts of fragrances, preservative, skin feel modifier (e.g., guar) etc. It may also contain free fatty acid and/or structurant/inert filler.

The surfactant system of the second chip preferably comprises either or both of the following ingredients:
(i) carboxylic acid soap;
(ii) synthetic anionic surfactant, preferably in the solid form at 25°C, such as sodium cocoyl isethionate, and an amphoteric surfactant such as cocoamidopropyl betaine.

In a further embodiment of the invention, the invention comprises a method of making benefit agent containing adjuvant compositions in the form of chips, flakes, granules, powders or mixtures thereof comprising:
(1) 50-95% of a carrier selected from group (a) (1)-(2) above;
(2) 5 to 50% benefit agents selected from group (b);
(3) 0-10% optional ingredients selected from thickeners and rheology modifiers;
(4) 0-10% water.

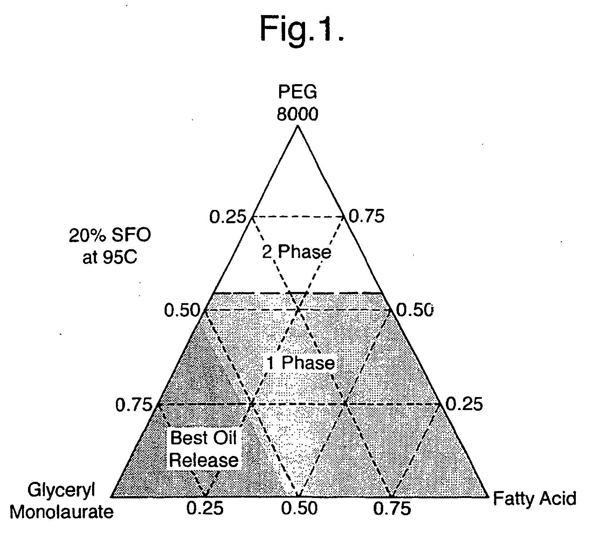

In the drawings, figure 1 presents the phase diagram of PEG 8000, fatty (stearic/palmitic) acid and polyol ester (glyceryl monolaurate) at 95°C. The ternary system contains sunflower seed oil at fixed level of 20% wt (i.e., the total concentration of PEG 8000, fatty acid and the glyceryl monolaurate equals to 80% total composition).

In one embodiment the present invention relates to novel personal washing bar compositions, particularly compositions in which the surfactant system comprises greater than 5%, preferably greater than 10% emollient oil and humectant.
The bar is primarily structured by a specific polyol ester with defined range of HLB and melting temperature.

Unexpectedly, the applicants have found that when the HLB of the solid polyol ester is between 2 and 15, preferably between 2.5 and 10, most preferably between 3 and 8, high levels of the emollient oil and/or humectant can be satisfactorily structured in the solid bar matrix, and the bar permits oil and/or humectant to be released to aqueous washing liquid to be delivered to the skin via personal wash.

In order to ensure that the oils are satisfactorily structured in the solid bar matrixes and can be released from the bar into aqueous personal wash liquor, the weight ratio of the polyol esters to the oil and/or humectants has to be greater than or equal to 1:1, preferably greater than or equal to 1.5:1, and most preferably greater than or equal to 2:1. For the same purpose, the weight ratio of the polyol esters to other optional structurants has to be above 1:1.

The compositions are defined in greater detail below:

### (a) Surfactant System

The surfactant system of the subject invention generally comprises 5% to 70%, preferably 10% to 60%, most preferably 15% to 40% total composition surfactant or mixtures of surfactants. The surfactants generally comprise anionic surfactants, amphoteric surfactants, nonionic surfactants, cationic surfactants and mixtures thereof, preferably anionic surfactants, amphoteric surfactants, nonionic surfactants and mixtures thereof.

### Anionic Surfactants

The anionic surfactant may be, for example, an aliphatic sulfonate, such as a primary alkane (e.g., C₈-C₂₂) sulfonate, primary alkane (e.g., C₈-C₂₂) disulfonate, C₈-C₂₂ alkene sulfonate, C₈-C₂₂ hydroxyalkane sulfonate or alkyl glyceryl ether sulfonate (AGS); or an aromatic sulfonate such as alkyl benzene sulfonate.

The anionic may also be an alkyl sulfate (e.g., C₁₂-C₁₈ alkyl sulfate) or alkyl ether sulfate (including alkyl glyceryl ether sulfates). Among the alkyl ether sulfates are those having the formula:

RO(CH₂CH₂0)ₙSO₃M

wherein R is an alkyl or alkenyl having 8 to 18 carbons, preferably 12 to 18 carbons, n has an average value of greater than 1.0, preferably between 2 and 3; and M is a solubilizing cation such as sodium, potassium, ammonium or substituted ammonium. Ammonium and sodium lauryl ether sulfates are preferred.

The anionic may also be alkyl sulfosuccinates (including mono- and dialkyl, e.g., C₆-C₂₂ sulfosuccinates); alkyl and acyl taurates, alkyl and acyl sarcosinates, sulfoacetates, C₈-C₂₂ alkyl phosphates and phosphates, alkyl phosphate esters and alkoxyl alkyl phosphate esters, acyl lactates, C₈-C₂₂ monoalkyl succinates and maleates, sulphoacetates, and acyl isethionates.

Sulfosuccinates may be monoalkyl sulfosuccinates having the formula:

R⁴O₂CCH₂CH(SO₃M)CO₂M;

amido-MEA sulfosuccinates of the formula

R⁴CONHCH₂CH₂O₂CCH₂CH(SO₃M)CO₂M

wherein R⁴ ranges from C₈-C₂₂ alkyl and M is a solubilizing cation;
amido-MIPA sulfosuccinates of formula

RCONH(CH₂)CH(CH₃)(SO₃M)CO₂M

where M is as defined above.

Also included are the alkoxylated citrate sulfosuccinates; and alkoxylated sulfosuccinates such as the following: 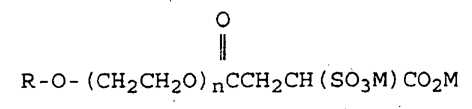 wherein n = 1 to 20; and M is as defined above.

Sarcosinates are generally indicated by the formula

RCON(CH₃)CH₂CO₂M,

wherein R ranges from C₈-C₂₀ alkyl and M is a solubilizing cation.

Taurates are generally identified by formula

R²CONR³CH₂CH₂SO₃M

wherein R² ranges from C₈-C₂₀ alkyl, R³ ranges from C₁-C₄ alkyl and M is a solubilizing cation.

Another class of anionics are carboxylates such as follows:

R-(CH₂CH₂O)ₙCO₂M

wherein R is C₈ to C₂₀ alkyl; n is 0 to 20; and M is as defined above.

Another carboxylate which can be used is amido alkyl polypeptide carboxylates such as, for example, Monteine LCQ ^{(R)} by Seppic.

Another surfactant which may be used are the C₈-C₁₈ acyl isethionates. These esters are prepared by reaction between alkali metal isethionate with mixed aliphatic fatty acids having from 6 to 18 carbon atoms and an iodine value of less than 20. At least 75% of the mixed fatty acids have from 12 to 18 carbon atoms and up to 25% have from 6 to 10 carbon atoms.

Acyl isethionates, when present, will generally range from about 0.5-15% by weight of the total composition. Preferably, this component is present from about 1 to about 10%.

The acyl isethionate may be an alkoxylated isethionate such as is described in Ilardi et al., U.S. Patent No. 5,393,466, hereby incorporated by reference into the subject application. This compound has the general formula: 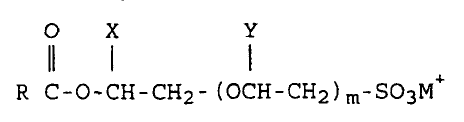 wherein R is an alkyl group having 8 to 18 carbons, m is an integer from 1 to 4., X and Y are hydrogen or an alkyl group having 1 to 4 carbons and M⁺ is a monovalent cation such as, for example, sodium, potassium or ammonium.

Another surfactant that may be used is the salts of C₈-C₂₄ carboxylic acid (soap). Preferably, for reducing the soap irritation to skin, the concentration of C₈-C₂₄ carboxylate fatty acid soap is at or less than 10% of total bar composition. More preferably, concentration of the soap is at or below 4% of total bar composition. Most preferably, the carboxylic acid soap is excluded from the bar composition of the subject invention.

### Zwitterionic and Amphoteric Surfactants

Zwitterionic surfactants are exemplified by those which can be broadly described as derivatives of aliphatic quaternary ammonium, phosphonium, and sulfonium compounds, in which the aliphatic radicals can be straight or branched chain, and wherein one of the aliphatic substituents contains from about 8 to about 18 carbon atoms and one contains an anionic group, e.g., carboxy, sulfonate, sulfate, phosphate, or phosphonate. A general formula for these compounds is: 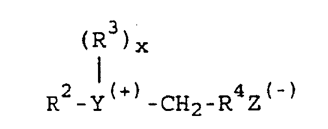 wherein R² contains an alkyl, alkenyl, or hydroxy alkyl radical of from about 8 to about 18 carbon atoms, from 0 to about 10 ethylene oxide moieties and from 0 to about 1 glyceryl moiety; Y is selected from the group consisting of nitrogen, phosphorus, and sulfur atoms; R³ is an alkyl or monohydroxyalkyl group containing about 1 to about 3 carbon atoms; X is 1 when Y is a sulfur atom, and 2 when Y is a nitrogen or phosphorus atom; R⁴ is an alkylene or hydroxyalkylene of from about 1 to about 4 carbon atoms and Z is a radical selected from the group consisting of carboxylate, sulfonate, sulfate, phosphonate, and phosphate groups.

Examples of such surfactants include:
4-[N,N-di(2-hydroxyethyl)-N-octadecylammonio]-butane-1-carboxylate;
5-[S-3-hydroxypropyl-S-hexadecylsulfonio]-3-hydroxypentane-1-sulfate;
3-[P,P-diethyl-P-3,6,9-trioxatetradexocylphosphonio]-2-hydroxypropane-1-phosphate;
3-[N,N-dipropyl-N-3-dodecoxy-2-hydroxypropylammonio]-propane-1-phosphonate;
3-(N,N-dimethyl-N-hexadecylammonio)propane-1-sulfonate;
3-(N,N-dimethyl-N-hexadecylammonio)-2-hydroxypropane-1-sulfonate;
4-[N,N-di(2-hydroxyethyl)-N-(2-hydroxydodecyl)ammonio]-butane-1-carboxylate;
3-[S-ethyl-S-(3-dodecoxy-2-hydroxypropyl)sulfonio]-propane-1-phosphate;
3-[P,P-dimethyl-P-dodecylphosphonio]-propane-1-phosphonate; and
5-[N,N-di(3-hydroxypropyl)-N-hexadecylammonio]-2-hydroxy-pentane-1-sulfate.

Amphoteric detergents which may be used in this invention include at least one acid group. This may be a carboxylic or a sulphonic acid group. They include quaternary nitrogen and therefore are quaternary amido acids. They should generally include an alkyl or alkenyl group of 7 to 18 carbon atoms. They will usually comply with an overall structural formula: 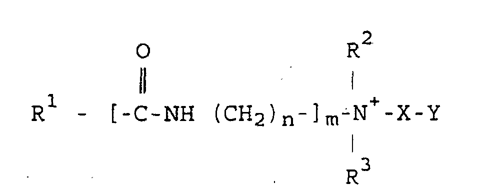
where R¹ is alkyl or alkenyl of 7 to 18 carbon atoms;
R² and R³ are each independently alkyl, hydroxyalkyl or carboxyalkyl of 1 to 3 carbon atoms;
n is 2 to 4;
m is 0 to 1;
X is alkylene of 1 to 3 carbon atoms optionally substituted with hydroxyl, and
Y is -CO₂- or -SO₃-

Suitable amphoteric detergents within the above general formula include simple betaines of formula: 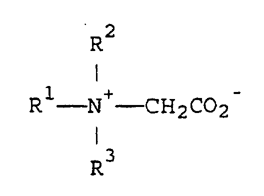 and amido betaines of formula: 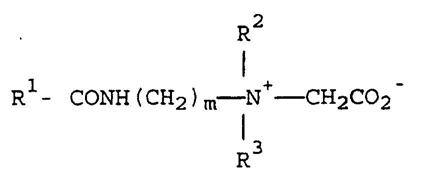 where m is 2 or 3.

In both formulae R¹, R² and R³ are as defined previously R¹ may in particular be a mixture of C₁₂ and C₁₄ alkyl groups derived from coconut so that at least half, preferably at least three quarters of the groups R¹ have 10 to 14 carbon atoms. R² and R³ are preferably methyl.

A further possibility is that the amphoteric detergent is a sulphobetaine of formula 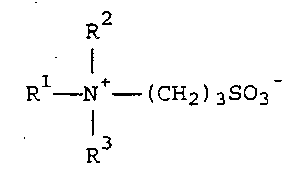 or 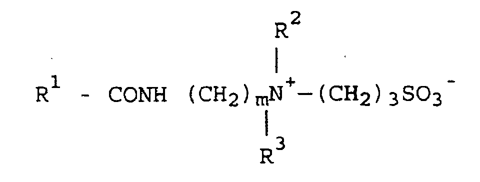 where m is 2 or 3, or variants of these in which -(CH₂)₃SO⁻₃ is replaced by

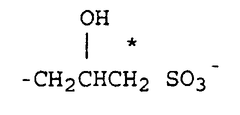

In these formulae R¹, R² and R³ are as discussed previously.

A further possibility is that the amphoteric detergent is a sulphobetaine of formula 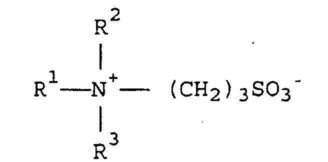 or 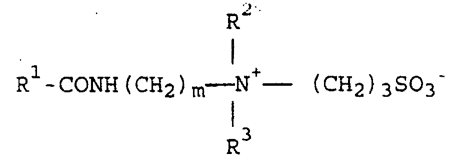 where m is 2 or 3, or variants of these in which -(CH₂)₃SO₃⁻ is replaced by

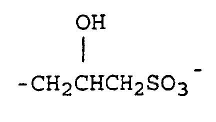

In these formulae R¹, R² and R³ are as discussed previously.

Amphoacetates and diamphoacetates are also intended to be covered in possible zwitterionic and/or amphoteric compounds which may be used.

The amphoteric/zwitterionic generally comprises 0.1 to 20% by weight, preferably 0.1% to 15%, more preferably 0.1 to 10% by wt. of the composition.

In addition to one or more anionic and amphoteric and/or zwitterionic, the surfactant system may optionally comprise a nonionic surfactant.

### Nonionic Surfactants

The nonionic which may be used includes in particular the reaction products of compounds having a hydrophobic group and a reactive hydrogen atom, for example aliphatic alcohols, acids, amides or alkyl phenols with alkylene oxides, especially ethylene oxide either alone or with propylene oxide. Specific nonionic detergent compounds are alkyl (C₆-C₂₂) phenols-ethylene oxide condensates, the condensation products of aliphatic (C₈-C₁₈) primary or secondary linear or branched alcohols with ethylene oxide, and products made by condensation of ethylene oxide with the reaction products of propylene oxide and ethylenediamine. Other so-called nonionic detergent compounds include long chain tertiary amine oxides, long chain tertiary phosphine oxides and dialkyl sulphoxides.

The nonionic may also be a sugar amide, such as a polysaccharide amide. Specifically, the surfactant may be one of the lactobionamides described in U.S. Patent No. 5,389,279 to Au et al. which is hereby incorporated by reference or it may be one of the sugar amides described in Patent No. 5,009,814 to Kelkenberg, hereby incorporated into the application by reference.

Other surfactants which may be used are described in U.S. Patent No. 3,723,325 to Parran Jr. and alkyl polysaccharide nonionic surfactants as disclosed in U.S. Patent No. 4,565,647 to Llenado, both of which are also incorporated into the application by reference.

Preferred alkyl polysaccharides are alkylpolyglycosides of the formula

R²O(CₙH₂ₙO)ₜ(glycosyl)ₓ

wherein R² is selected from the group consisting of alkyl, alkylphenyl, hydroxyalkyl, hydroxyalkylphenyl, and mixtures thereof in which alkyl groups contain from about 10 to about 18, preferably from about 12 to about 14, carbon atoms; n is 0 to 3, preferably 2; t is from 0 to about 10, preferably 0; and x is from 1.3 to about 10, preferably from 1.3 to about 2.7. The glycosyl is preferably derived from glucose. To prepare these compounds, the alcohol or alkylpolyethoxy alcohol is formed first and then reacted with glucose, or a source of glucose, to form the glucoside (attachment at the 1-position). The additional glycosyl units can then be attached between their 1-position and the preceding glycosyl units 2-, 3-, 4- and/or 6-position, preferably predominantly the 2-position.

### (b) Emollient Oils and Humectants

The personal washing bar also contains 5% to 40%, preferably 10% to 25% of the total bar composition of a liquid hydrophobic emollient oil, a liquid hydrophilic humectant or mixtures thereof.

The liquid hydrophobic emollient oil has a solubility of less than 10%, preferably less than 5%, and most preferably less than 1% in water at 25°C.

The liquid emollient oil has a melting temperature of less than 25°C and has a viscosity less than 10⁵ centipoise, preferably less than 50,000 centipoise, most preferably less than 10,000 centipoise at 25°C. The defined melting temperature and viscosity range of said oil is a criticality since keeping the oil in a free flow liquid state is important for satisfactory bar mixing as well as for pouring into the bar mold when preferred cast melt process is applied. For example, above the viscosity range, oil becomes very thick, and this prevents the efficiently mixing of the bar ingredients at molten state (e.g., 85-125°C), reduces the pourability of the melt, and causes bar in homogeneity and processing difficulties.

The emollient oil is selected from hydrocarbon oils, silicones, liquid diglycerides, liquid triglycerides, liquid di- and tri-glyceride derivatives, liquid hydrocarbon esters, silicones, sterols, lanolins and sunscreen oils, and mixtures thereof.

Examples of hydrocarbon oils are mineral oil, petrolatum, C8-C24 straight or branched chain alkyl or alkenyl compounds.

Examples of liquid di- and tri-glycerides and their derivatives are sorbitol, coconut oil, jojoba oil, maleated soybean oil, castor oil, almond oil, peanut oil, wheat germ oil, rice bran oil, linseed oil, apricot pits oil, walnuts, palm nuts, pistachio nuts, sesame seeds, rape seed oil, cade oil, corn oil, peach pit oil, poppyseed oil, pine oil, soybean oil, avocado oil, sunflower seed oil, hazelnut oil, olive oil, grapeseed oil, and safflower oil, Shea butter, babassu oil, milk glycerides and mixtures thereof.

Examples of silicone oil include dimethicone copolyol, and dimethylpolysiloxane.

Examples of hydrocarbon esters include isopropyl myristate and isocetyl palmitate.

Examples of the sunscreen oils include butyl methoxydibenzoylmethane (tradename: Parsol 1789), octyl methoxy cinnamate (tradename: Parsol MCX), benzophenone quat, niacinamide, padimate 0, P-proline.

It is more preferred that the emollient oil is selected from liquid di- and tri-glycerides and their derivatives.

The liquid hydrophilic humectant , when used, has a solubility of greater than or equal to 50% wt. in water at 25°C.

The liquid humectant has a melting temperature at less than 25°C and has a viscosity at less than 5000 centipoise, preferably less than 1000 centipoise.

The liquid humectant is selected from polyols consisting of glycerol, glycerin, propylene glycol, liquid polyalkylene glycols such as polypropylene glycols, polyethylene glycols with molecular weight less than 1000 (such that they are in liquid state at 25°C), ethyl hexanediol, and hexylene glycols.

### (c) Solid Amphiphilic Polyol Esters

The bar of the present invention also comprises 15% to 70%, preferably 20% to 50%, of the total composition of a solid amphiphilic polyol ester.

The amphiphilic polyol ester is specified by its hydrophilic lipophilic balance (HLB) value that is defined by Becher and Schick and by Marszall in Chapter 8 and Chapter 9 of Nonionic Surfactants - Phase Chemistry, Surfactant Sci. Series, Vol. 23, P439-549, which is hereby incorporated by reference into the subject application as reference. The solid, amphiphilic polyol ester has a hydrophilic-lipophilic balance (HLB) value between 2 and 15, preferably between 2.5 and 10, and most preferably between 3 and 8. The HLB range of the polyol ester is a criticality because below the range, the polyol ester can bind too tightly with the oil (b) and not allow the oil to be released to the aqueous solution, which prevents the oil to be delivered to the skin; and above the HLB range, the polyol ester does not have the capability to structure the emollient oil and the humectant described in (b) in the solid bar matrix and causes oil leakage and separation from the bulk.

The polyol ester has a melting temperature between 40°C and 90°C, preferably at between 45°C and 70°C; such that the matrix formed by the polyol ester to trap the oil is in the solid form in the bar under in-use condition.

The weight ratio of the polyol ester to the sum of the emollient oil and the humectants, both described in (b), is greater than or equal to 1:1, preferably greater than or equal to 1.5:1; this weight ratio is a criticality because below this ratio, the oil and the humectant cannot be satisfactorily contained in the solid matrix of the bar, which leads to oil leakage and separation from the bulk.

The solid amphiphilic polyol ester is defined as a polyol esterified or partially esterified by an organic acid that can be represented by 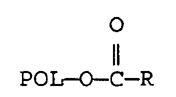 in which POL is a polyol moiety, R is a hydrophobic moiety, and one or more {-O-(C=O)-R} functional groups are chemically attached to one or more hydroxy groups of the polyol moiety.

For example, the polyol moiety (POL) may be derived from glycerol, glycerin, propylene glycol, polypropylene glycols, ethylene glycol, polyethylene glycols, ethyl hexanediol, hexylene glycols and pentaeryrthrityl or mixtures thereof.

The hydrophobic group R is selected from the derivatives of alkyl, aryl, alkylaryl, alkylene, acyl, and fat and oil derivatives or mixtures thereof. Preferably R is the derivative of a C8-C22 straight or branched chain alkyl functioning group, most preferably a C12-C22 alkyl functioning group.

Examples of said solid, amphiphilic polyol ester include glycerin fatty esters and glycerol esters, such as glyceryl monolaurate (from Henkel under the tradename of Monomuls 90L-12) and glyceryl monostearate (from Stepan under the tradename of GMS Pure); alkylene glycol fatty esters, such as ethylene glycol monostearate and ethylene glycol monolaurate (from RP under the tradename of Alkamuls); pentaeryrthrityl fatty esters such as pentaeryrthrityl stearate; polyglycerin fatty esters such as hexaglyceryl tristearate. The physical properties of a few suitable polyol esters were listed in Table 1.

**Table 1**

| Examples and properties of the suitable polyol esters for the present invention: | | | |
|---|---|---|---|
| Polyol Ester | Melting temperature (°C) | HLB Value | Tradename /supplier |
| Glyceryl monolaurate | 56-65 | 4.9 | Monomuls 90L-12 / Henkel Corp. |
| Glyceryl monostearate | 56-65 | 3.8 | Kessco GMS Pure / Stepan |
| Ethylene glycol stearate | 52-56 | 2.9 | Kessco EGMS 70/ Stepan |

### (d) Optional Structurants

The compositions may also contain 0 to 30% by wt., preferably 5 to 20% by wt. of an optional structurant and/or filler. Such structurants can be used to enhance the bar integrity, improve the processing properties, and enhance desired user sensory profiles.

The total weight percentage of the optional structurants and/or filler has to be less than the weight percentage of the polyol ester defined in (c). This specification on the upper limit of the optional structurants is a criticality because above this range, the bar has its oil structuring capability reduced, which causes oil leakage and/or oil phase separation from the bulk; or the bar has its oil releasing capability reduced, which prevents the oil release into the aqueous washing liquor and deliver to the skin via the personal washing route.

The optional structurant is generally long chain, preferably straight and saturated, (C₈-C₂₄) fatty acid or ester derivative thereof; and/or branched long chain, preferably straight and saturated, (C₈-C₂₄) alcohol or ether derivatives thereof.

The optional structurant can also be polyalkylene glycol with molecular weight between 2000 and 20,000, preferably between 3000 and 10,000. Those PEGs are commercially available, such as those marketed under the tradename of CARBOWAX SENTRY PEG 8000 or PEG 4000 by Union Carbide.

The optional structurants that can be used include starches, preferably water soluble starches such as maltodextrin and polyethylene wax or paraffin wax.

The optional structurant can also be selected from water soluble polymers chemically modified with hydrophobic moiety or moieties, for example, EO-PO block copolymer, hydrophobically modified PEGs such as POE(200)-glyceryl-stearate, glucam DOE 120 (PEG 120 Methyl Glucose Dioleate), and Hodag CSA-102 (PEG-150 stearate), and Rewoderm ^{(R)} (PEG modified glyceryl cocoate, palmate or tallowate) from Rewo Chemicals.

The optional structurants also include Amerchol Polymer HM 1500 (Nonoxynyl Hydroethyl Cellulose).

In addition, the bar compositions of the invention may include 0 to 15% by wt. optional ingredients as follows:
perfumes; sequestering agents, such as tetrasodium ethylenediaminetetraacetate (EDTA), EHDP or mixtures in an amount of 0.01 to 1%, preferably 0.01 to 0.05%; and coloring agents, opacifiers and pearlizers such as zinc stearate, magnesium stearate, TiO₂ EGMS (ethylene glycol monostearate) or Lytron 621 (Styrene/Acrylate copolymer); all of which are useful in enhancing the appearance or cosmetic properties of the product.

The compositions may further comprise antimicrobials such as 2-hydroxy-4,2'4' trichlorodiphenylether (DP300); preservatives such as dimethyloldimethylhydantoin (Glydant XL1000), parabens, sorbic acid etc.

The compositions may also comprise coconut acyl mono- or diethanol amides as suds boosters, and strongly ionizing salts such as sodium chloride and sodium sulfate may also be used to advantage.

Antioxidants such as, for example, butylated hydroxytoluene (BHT) may be used advantageously in amounts of about 0.01% or higher if appropriate.

Cationic polymers as conditioners which may be used include Quatrisoft LM-200 Polyquaternium-24, Merquat Plus 3330-Polyquaternium 39; and Jaguar ^{(R)} type conditioners.

Polyethylene glycols as conditioners which may be used. include:
Polyox WSR-205 PEG-14M,
Polyox WSR-N-60K PEG 45M, or
Polyox WSR-N-750 PEG 7M.

Another ingredient which may be included are exfoliants such as polyoxyethylene beads, walnut shells and apricot seeds.

Another ingredient which may be included are 0-20% zinc oxides and titanium oxide for the purpose of protecting the skin from sun damage.

In another embodiment, the invention relates to novel personal washing bar compositions comprised of (1) adjuvant chips containing skin benefit agents and (2) base chips containing a surfactant system.

Particularly, the adjuvant chips are made containing a specific polyol ester with defined HLB and defined melting temperature as the major carrier for liquid hydrophobic oils and/or hydrophilic humectants. Unexpectedly, the applicants have found that when the HLB of the solid polyol ester is between 2.5 and 15, preferably between 3 and 8, high levels of the emollient oil and the humectant can be satisfactorily structured in the solid matrix, and the solid matrix permits oil and humectant to be release to aqueous washing liquid to be delivered to the skin via personal wash.

The invention further comprises an extruded bar composition which is produced using about 5 to 80%, preferably 10 to 50%, most preferably 20% to 40% of the adjuvant composition in the form of solid chips, flakes, powders, granules or mixtures thereof; and about 20 to 95% of a surfactant system (base chips) in the form of chips, flakes, granules or mixtures thereof, wherein the surfactant is selected from anionic surfactants, amphoteric surfactants, nonionic surfactants, cationic surfactants and mixtures thereof. The surfactant system may also contain minor amounts of fragrances, preservative, skin feel modifier (e.g., guar) etc. It may also contain free fatty acid and/or structurant/inert filler.

The surfactant system of the second chip preferably comprises either or both of the following ingredients:
(i) carboxylic acid soap;
(ii) synthetic anionic surfactant, preferably in the solid form at 25°C, such as sodium cocoyl isethionate, and an amphoteric surfactant such as cocoamidopropyl betaine.

In yet another embodiment of the invention, the invention comprises a method of making benefit agent containing adjuvant compositions in the form of chips, flakes, granules, powders or mixtures thereof comprising:
(1) 50-95% of a carrier selected from group (a) (1)-(2) above;
(2) 5 to 50% benefit agents selected from group (b) ;
(3) 0-10% optional ingredients selected from thickeners and rheology modifiers;
(4) 0-10% water.

The adjuvant chips comprise 50-95%, preferably 65% to 90% of adjuvant chip of the same amphiphilic polyol ester described in connection with the bar compositions above. They may also contain optional structurants same as discussed above in connection with the bar composition. In the chips, structurants can be used to improve processing properties, enhance desired sensory profiles, and to modify dissolution rates of the adjuvant chips to enhance bar integrity.

The total weight percentage of the optional structurant and/or filler has to be less than the weight percentage of the polyol ester in the adjuvant chip composition. This specification on the upper limit of the optional structurants is a criticality because above this range, the chip composition has its oil structuring capability reduced, which causes oil leakage and/or oil phase separation from the bulk; or the bar has its oil releasing capability reduced, which prevents the oil release into the aqueous washing liquor and deliver to the skin via the personal washing route.

The adjuvant chips also contain 5% to 50%, preferably 10% to 35%, most preferably 10% to 25% of the total bar composition of a liquid hydrophobic emollient oil, a liquid hydrophilic humectant or mixtures thereof.

The liquid hydrophobic emollient oil has a solubility of less than 10%, preferably less than 5%, and most preferably less than 1% in water at 25°C.

The liquid emollient oil has a melting temperature of less than 25°C and has a viscosity less than 10⁵ centipoise, preferably less than 50,000 centipoise, most preferably less than 10,000 centipoise at 25°C. The defined melting temperature and viscosity range of said oil is a criticality since keeping the oil in a free flow liquid state is important for satisfactory bar mixing as well as for pouring into the bar mold when preferred cast melt process is applied. For example, above the viscosity range, oil becomes very thick, and this prevents the efficiently mixing of the bar ingredients at molten state (e.g., 85-125°C), reduces the pourability of the melt, and causes bar in homogeneity and processing difficulties.

The emollient oil is selected from hydrocarbon oils, silicones, liquid diglycerides, liquid triglycerides, liquid di- and tri-glyceride derivatives, liquid hydrocarbon esters, silicones, sterols, lanolins and sunscreen oils. Examples of hydrocarbon oils are mineral oil, petrolatum, C8-C24 straight or branched chain alkyl or alkenyl compounds.

Examples of liquid di- and tri-glycerides and their derivatives are sorbitol, coconut oil, jojoba oil, maleated soybean oil, castor oil, almond oil, peanut oil, wheat germ oil, rice bran oil, linseed oil, apricot pits oil, walnuts, palm nuts, pistachio nuts, sesame seeds, rape seed oil, cade oil, corn oil, peach pit oil, poppyseed oil, pine oil, soybean oil, avocado oil, sunflower seed oil, hazelnut oil, olive oil, grapeseed oil, and safflower oil, Shea butter, babassu oil, milk glycerides and mixtures thereof.

Examples of silicone oil include dimethicone copolyol, and dimethylpolysiloxane.

Examples of hydrocarbon esters include isopropyl myristate and isocetyl palmitate.

Examples of the sunscreen oils include UV-absorbing oils selected from the group consisting of butyl methoxydibenzoylmethane (tradename: Parsol 1789), PABA, octyl methoxy cinnamate (tradename: Parsol MCX), benzophenone quat, niacinamide, padimate 0, P-proline.

It is more preferred that the emollient oil is selected from liquid di- and tri-glycerides and their derivatives.

The liquid hydrophilic humectant, when used, has a solubility of greater than or equal to 50% wt. in water at 25°C.

The liquid humectant has a melting temperature at less than 25°C and has a viscosity at less than 5000 centipoise, preferably less than 1000 centipoise.

The humectant is selected from polyols consisting of glycerol, glycerin, propylene glycol, liquid polyalkylene glycols such as polypropylene glycols, polyethylene glycols with molecular weight less than 1000 (such that they are in liquid state at 25°C), ethyl hexanediol, and hexylene glycols.

In addition, the adjuvant chip composition of the invention may include 0 to 15% by wt. optional ingredients as follows:
perfumes; sequestering agents, such as tetrasodium ethylenediaminetetraacetate (EDTA), EHDP or mixtures in an amount of 0.01 to 1%, preferably 0.01 to 0.05%; and coloring agents, opacifiers and pearlizers such as zinc stearate, magnesium stearate, TiO₂, EGMS (ethylene glycol monostearate) or Lytron 621 (Styrene/Acrylate copolymer); all of which are useful in enhancing the appearance or cosmetic properties of the product.

The chip composition may further comprise antimicrobials such as 2-hydroxy-4,2'4' trichlorodiphenylether (DP300) ; preservatives such as dimethyloldimethylhydantoin (Glydant XL1000), parabens, sorbic acid etc.

The chip composition may also comprise coconut acyl mono- or diethanol amides as suds boosters, and strongly ionizing salts such as sodium chloride and sodium sulfate may also be used to advantage.

Antioxidants such as, for example, butylated hydroxytoluene (BHT) may be used advantageously in amounts of about 0.01% or higher if appropriate.

Cationic polymers as conditioners which may be used include Quatrisoft LM-200 Polyquaternium-24, Merquat Plus 3330-Polyquaternium 39; and Jaguar ^{(R)} type conditioners.

The chip composition may also comprise 0-10% of the total chip composition a thickening agent selected from silicas, starches or mixture of both. Preferably, said starches are maltodextrin or potato or corn starch. A referred silica is fumed silica, generally produced by the hydrolysis of silicon tetrachloride vapor in a flame of hydrogen and oxygen. The process produces particles of from 7 to 30 millimicrons. Preferably, said thickeners are incorporated in the chip under the condition that said optional structurants (defined in (1)) are included in the chip composition.

Finally, the adjuvant chips may be mixed with chips comprising surfactant system ("base" chip).

The base chip comprises 10% to 70%, preferably 15% to 60%, most preferably 25% to 50% total chip composition surfactant or mixture of surfactants the same as the surfactant system described above relating to bar compositions.

The base chips may also comprise an optional structurant and/or filler. Such structurants can be used to improve the processing properties, and enhance desired user sensory profiles, and modify the melting temperature, Krafft temperature, and dissolution rates of the base chips to enhance bar's integrity.

The structurant is as described above in connection with the bar composition.

The base chip may also optionally include 0 to 15% ingredients same as those discussed in the bar composition above.

The present invention is set forth in greater detail in the examples which follow. The examples are for illustration purposes only and are not intended to limit the scope of the claims in any way.

Except in the operating and comparative examples, or where otherwise explicitly indicated, all numbers in this description indicating amounts or ratios of materials or conditions of reaction, physical properties of materials and/or use are to be understood as modified by the word "about."

The use of the word "comprising" in this specification is intended to specify presence of stated features, steps, components etc., but does not preclude presence or addition of one or more features, integers, steps, components or groups thereof.

All percentages in the examples and specification, unless indicated otherwise, are intended to be percentages by weight.

### EXAMPLES

### Protocol

### Mildness Assessments:

Zein dissolution test was used to preliminary screen the irritation potential of the formulations studied. In an 8 oz. jar, 30 mLs of an aqueous dispersion of a formulation were prepared. The dispersions sat in a 45°C bath until fully dissolved. Upon equilibration at room temperature, 1.5 gms of zein powder were added to each solution with rapid stirring for one hour. The solutions were then transferred to centrifuge tubes and centrifuged for 30 minutes at approximately 3,000 rpms. The undissolved zein was isolated, rinsed and allowed to dry in a 60°C vacuum oven to a constant weight. The percent zein solubilized, which is proportional to irritation potential, was determined gravimetrically.

The 3-4 Day Patch Test was used to evaluate skin mildness of aqueous dispersions containing 1% DEFI active (sodium cocoyl isethionate) and different levels of the structurant/coactives. Patches (Hilltop^{(R)}. Chambers, 25 mm in size) were applied to the outer upper arms. of the panelists under bandage type dressings (Scanpor^{(R)} tape). After each designated contact periods (24 hrs. for the first patch application, 18 hrs. for the second, third or fourth applications), the patches were removed and the sites were visually ranked in order of severity (erythema and dryness) by trained examiners under consistent lighting.

### Formulation Processing:

Bar solids were prepared by a cast melt process. First, the components were mixed together at 80-120°C in 100g to 2000g scale in a liquid mixer for 30-60 minutes using an overhead stir. Then rest of the components were added, and the water level was adjusted to approximately 8-15 wt %. The batch was covered to prevent moisture loss and was mixed for about 15-45 minutes. Then the cover was removed, and the mixture was allowed to dry. The moisture content of the samples was taken at different times during the drying stage and was determined by Karl Fisher titration with a turbo titrator. At the final moisture level (0-3%), the mixture in the form of free-flow liquid was dropped to bar molds and was allowed to be cooled at room temperature for 4 hours. Upon solidification, the molten mixture in the mold was casted into bar solids.

### EXAMPLE 1

### Advantages of Using Polyol Ester as Oil Structurant in Comparison with PEG 8000 and Palmitic/stearic Acid

Carrying 20% sunflower seed oil, a bar structuring system comprised of Polyol ester (glyceryl monolaurate), PEG 8000 and fatty acid was selected to test the formulation space for satisfactory oil structuring and releasing capabilities.

### Comparative 1

Shown in the ternary phase diagram (Figure 1), samples containing high levels of PEG 8000 (i.e., concentration of PEG 8000 is above 50% total structuring system) separated into an oily top layer and a bottom layer comprised of the rest. Cooling the PEG-rich samples to room temperature resulted in tacky solids with oil leaking out. This implies that PEG 8000 is not suitable as the major structurant for a high oil bar, which is consistent with the findings discussed in Example 2.

### Comparative 2

In the fatty acid rich region of the Figure 1 (i.e., concentration of FA is above 60% total structuring system), samples formed single isotropic liquids at 95°C. Cooling those samples to 25°C resulted in firm, crisp solids. However, there was no oil released from the solids into water, as observed under optical microscopy, and this was not desired for benefit delivery. Thus the traditional hydrophobic binders, such as stearic/palmitic acid or wax are not ideal as the major structurants for the high oil bars.

### Invention

In the polyol ester rich region (i.e., glyceryl monolaurate concentration is above 50%), samples formed single-phase isotropic liquids at 95°C. Cooling the molten mixtures to 25°C resulted in firm, crisp solids, which permitted oil release into aqueous phase. Thus monoglyceride should be used as the major structurant (i.e., 50% and above of the total bar structuring system) for the optimum oil-carrying and releasing.

### EXAMPLE 2

### Formulations of Invention and Comparative Bars

Formulation F-1, F-2 and F-3 contains 20% sunflower seed oil and primarily structured by polyol esters such as glyceryl monolaurate and glyceryl monostearate (both are polyol esters). The major lathering ingredients in those formulations are SLES and CAP betaine. As a result, F-1 provides lotion like, oily lather, as well as strong after-wash moisturizing skin feel. F-2 provides the lather and skin feel similar to F-1, but the bar is significantly harder than F-1. F-3 provides the lather and skin feel similar to F-1 and F-2 but with significantly less mush. F-4 uses ethylene glycol monostearate (a polyol ester) to structure the bar to achieve a unique oily after-wash skin feel. F-5 contains sodium cocoyl isethionate as the major lathering surfactant. In combination with the sunflower seed oil and the monoglycerides, the bar F-5 provides more creamy, rich lather and retains the oily skin feel. Bar F-6 of the invention incorporated 20% glycerin instead of the sunflower seed oil. The polyol ester (glyceryl monostearate) structured the 20% glycerin liquid in the bar without leaking or phase separation, and the bar provides a unique moisturizing skin feel and is ultra-mild to the skin.

C-1 is a comparative bar containing no oil and no polyol ester, thus it lacks the oily feel provided by the bars of the subject invention (i.e., F1 through F-6). The bar is more irritative to skin when compared with the invention bars (see Example 3 and Example 4).

In contrast to the invention bars that are structured primarily by the polyol esters, C-2 is structured by PEG 8000 and stearic/palmitic (fatty) acid, and the weight ratio of glyceryl monolaurate to the sum of PEG 8000 and fatty acid is slightly less than 1 (i.e., 26.84/(25+3.67) = 0.94). As a result, a portion of the sunflower seed oil separated from the bulk during mixing, and oil pooling was found in the finished bar. Therefore, it is a criticality of the subject invention that the polyol ester has to be used as the primary structurant to trap the oil in the solid matrix of the bar.

**Table 2**

| Formulations of the invention and comparative bars | | | | | | | | |
|---|---|---|---|---|---|---|---|---|
| Ingredients | F-1 wt% | F-2 wt% | F-3 wt% | F-4 wt% | F-5 wt% | F-6 wt% | C-1 wt% | C-2 wt% |
| Sodium Cocoyl isethionate | 5.0 | 8.0 | 8.0 | 8.0 | 27.0 | 0 | 27.0 | 12.5 |
| Sodium lauryl sulfate | 10.0 | 6.0 | 6.0 | 6.0 | 0 | 10.0 | 0 | 5.0 |
| Cocoamidopropyl betaine | 10.0 | 6.0 | 6.0 | 6.0 | 5.0 | 10.0 | 5.0 | 5.0 |
| Glyceryl monolaurate | 19.0 | 40.65 | 0 | 0 | 27.6 | 0 | 0 | 26.84 |
| Glyceryl monostearate | 19.0 | 0 | 40.65 | 0 | 0 | 41.0 | 0 | 0 |
| Stearic/palmitic acid | 5.0 | 2.35 | 2.35 | 0 | 8.4 | 5.0 | 17.0 | 3.67 |
| Ethylene glycol monostearate | 0 | 0 | 0 | 40.65 | 0 | 0 | 0 | 0 |
| PEG 8000 | 8.0 | 15.0 | 15.0 | 15.0 | 10.0 | 10.0 | 22.0 | 25.0 |
| Maltodextrin | 0 | 0 | 0 | 0 | 0 | 0 | 10.0 | 0 |
| PEG 300 | 0 | 0 | 0 | 0 | 0 | 0 | 2.0 | 0 |
| PEG 1450 | 0 | 0 | 0 | 0 | 0 | 0 | 3.0 | 0 |
| Sodium stearate | 4.0 | 0 | 0 | 0 | 0 | 0 | 6.0 | 0 |
| Sodium isethionate | 0.2 | 0.4 | 0.4 | 0.4 | 1.2 | 0 | 2.2 | 0.5 |
| Glycerin | 0 | 0 | 0 | 0 | 0 | 20.0 | 0 | 0 |
| Sunflower seed oil | 20.0 | 20.0 | 20.0 | 20.0 | 20.0 | 0 | 0 | 20.0 |
| Water | | 1.6 | 1.6 | 1.6 | 0.8 | 4.0 | 5.8 | 1.5 |

### EXAMPLE 3

### Ultra Skin Mildness of the Bar Containing High Level of Oil and the Polyol Esters

Table 3 shows the weight percent of the Zein protein dissolved by cleansing bar formulations shown in Table 2, Example 2. Formulation F-1 and F-5 (invention) were found to dissolve no detectable amount of zein (<< 10%); in contrast, Formulation C-1 (comparative) dissolved about 16% Zein protein. The results show that the bars of the invention (F-1 & F-5) containing a high level of sun flower seed oil (i.e. 20%) and monoglycerides (a polyol ester) have ultra low irritation potential to skin. The comparative bar C-1 w/o the oil and the polyol ester may have a significantly higher skin irritation potential when compared with the F-1 and F-5 bars of the invention.

**Table 3**

| The Results of the Zein Testing | |
|---|---|
| Bar Formulation | Zein Protein % Dissolved |
| F-1 (Invention) | <<10 |
| F-5 (Invention) | <<10 |
| C-1 (Comparative) | 16.0 |

### EXAMPLE 4

As shown in Table 4, In-vivo Human Skin Patch Testing indicates that the bar of invention (i.e., F-1 and F-6 of Example 2) is significantly milder than the comparative bar (i.e., C-1 of Example 2). Not to be bound by the theory, the invention bar F-1 and F-6 contains high level of emollient oil or humectant and the monoglyceride (also an emollient; which in combination contribute to the skin mildness. Also the monoglyceride (a polyol ester) also structured liquid ultra mild surfactants such as SLES and CAP betaine in the bar, which reduced the skin irritation.

**Table 4**

| In-vivo Human Skin Patch Test Results | |
|---|---|
| Bar Formulation | Patch Test Score: Sum of Ranks at Day-4 (lower sum --> more irritative, a difference of approximately 18 in the sum of ranks is necessary for a test material to be significantly different from the control material (i.e., 90% confidence level)). |
| F-1 (Invention) | 52.0 |
| F-6 (Invention) | 81.0 |
| C-1 (comparative control) | 28.0 |

### EXAMPLE 5

The high oil bars developed in the subject invention can be used to carry sun-protecting oils, and the sun-protecting oils can be delivered from the bars to skin via skin cleansing. In formulation F-7 and F-8, Parsol MCX (a UV absorbing oil, sun protecting agent) is incorporated. Both bars provide oily skin feel. F-7 provides a lotion like lather, and F-8 provides creamy and rich lather.

| FULL CHEMICAL NAME OR CTFA NAME | F-7 % wt. | F-8 % wt. |
|---|---|---|
| Sodium cocoyl isethionate | 7.93 | 27.0 |
| Palmitic/Stearic acid | 2.44 | 8.68 |
| Cocamidopropyl Betaine | 5.95 | 5.00 |
| Ethylhexyl p-methoxycinnamate or (Parsol MCX) | 19.83 | 20.0 |
| PEG 8000 | 14.87 | 10.0 |
| Glycerol monostearate | 40.3 | 26.6 |
| sodium lauryl ether sulfate-3eo | 5.95 | --- |
| target Water | 1.0 | 0.57 |
| Fragrance(Dove Shower Gel) | 0.5 | 0.29 |
| Sodium isethionate | 0.49 | 1.15 |
| Sodium chloride | 0.4 | 0.37 |
| Titanium dioxide | 0.3 | 0.30 |
| EDTA | 0.02 | 0.02 |
| EHDP | 0.02 | 0.02 |
| Total | 100 | 100 |

### EXAMPLE 6

### Advantages of Using Polyol Ester as Oil Structurant in Comparison with PEG 8000 and Palmitic/stearic Acid

Carrying 20% sunflower seed oil, a bar structuring system comprised of Polyol ester (glyceryl monolaurate), PEG 8000 and fatty acid was selected to test the formulation space for satisfactory oil structuring and releasing capabilities.

### Comparative

Shown in the ternary phase diagram (Figure 1), samples containing high levels. of PEG 8000 (i.e., concentration of PEG 8000 is above 50% total structuring system) separated into an oily top layer and a bottom layer comprised of the rest. Cooling the PEG-rich samples to room temperature resulted in tacky solids with oil leaking out. This implies that PEG 8000 is not suitable as the major structurant for a high oil bar, which is consistent with the findings discussed in Example 2.

### Comparative

In the fatty acid rich region of the Figure 1 (i.e., concentration of FA is above 60% total structuring system), samples formed single isotropic liquids at 95°C. Cooling those samples to 25°C resulted in firm, crisp solids. However, there was no oil released from the solids into water, as observed under optical microscopy, and this was not desired for benefit delivery. Thus the traditional hydrophobic binders, such as stearic/palmitic acid or wax are not ideal as the major structurants for the high oil bars.

### Invention

In the polyol ester rich region (i.e., glyceryl monolaurate concentration is above 50%), samples formed single-phase isotropic liquids at 95°C. Cooling the molten mixtures to 25C resulted in firm, crisp solids, which permitted oil release into aqueous phase. Thus monoglyceride should be used as the major structurant (i.e., 50% and above of the total bar structuring system) for the optimum oil-carrying and releasing.

### EXAMPLE 7

### Preparation of Oil-Containing Adjuvant Chips

Adjuvant Chips were prepared by first melting 1500 grams of glycerol monostearate (from Stepan, under tradename of GMS pure) at temperatures between 85°C and 120°C using an overhead mixer for 30-120 minutes and allowing the GMS to deaerate. Then sunflower seed oil were stirred in. Upon melting and homogenous mixing, glycerol monostearate and sunflower seed oil became miscible with each other and formed an isotropic solution. Then the isotropic solution was gradually poured onto a chill roll with temperatures set between 0 to 15°C and collected as adjuvant chips or flakes. The adjuvant chips contain 30% sunflower seed oil and 70% glycerol monostearate and have melting temperatures between 50 and 70°C.

### EXAMPLE 8

### Preparation of a Finished Bar Containing Dove ^{(R)}

857 grams of the adjuvant chips (containing 30% sunflower seed oil) prepared by Example 6 were combined with 2000 grams of Dove ^{(R)} as base chips containing a surfactant system (representing 70% of final bar) in a Ribbon blender were plodded under vacuum in a Weber Seelander duplex refiner with screw speed at about 20 rpm. The nose cone of the plodder was heated to 45-50°C. The cut billets were stamped into bars using a Weber Seelander L4 hydraulic press with a nylon, pillow-shaped die in place.

The Finished bar contains 70% Dove ^{(R)} as the base chips and 30% said adjuvant chips. Said Dove ^{(R)} base chips have the following composition:
about 40-60% fatty acid isethionate;
about 20-30% fatty acid;
about 1-10% sodium isethionate;
about 5% cocoamidopropyl betain; and
remainder preservative, dyes, water, and other minors.

Plodding throughput rate was as good as Dove ^{(R)} alone. The experiments show that the emollient containing chips can be successfully incorporated into bars without affecting the processing, and thus the emollients (in this case, sunflower seed oil can be subsequently delivered. The bar also provided interesting sensory cues including creamy, dense lather, and oily moisturized skin after-wash.

### EXAMPLE 9

### Preparation of a Finished Bar Containing 82/18 Fatty Acid Soap

30% adjuvant chips, containing 30% sunflower seed oil, prepared by Example 6 were combined with 82/18 fatty acid soap as base chips, representing 70% of the final bar. The 82/18 fatty acid soap was first heated in a sigma blade mixer until the material became soft and pliable. The moisture was adjusted so as to have the final product containing 10%-13% moisture. At this time perfume was also added so as to have the final product containing 1.5% perfume. Then the fatty acid soap chips were refined into 3 mm diameter pellets and mixed in a bowl with the adjuvant chips. The blend was then re-refined into 3 mm diameter pellets to insure homogeneity of the 82/18 soap and the adjuvant chips. Further processing produced extruded billets which were cut and stamped into bars. No point of the process was hindered by the addition of adjuvant chips to the soap base.

## Claims

1. A solid skin cleansing bar comprising:
(a) 5% to 70% by weight of the total bar composition surfactant or mixtures of surfactants; and
(b) 5% to 40% by weight of the total bar composition of a liquid hydrophobia emollient oil or a liquid humectant or mixtures thereof;
the liquid hydrophobic emollient oil having a water solubility less than 10% by weight in water at 25°C; said liquid emollient oil having a melting temperature of less than 25°C; said oil having a viscosity less than 10⁵ centipoise at 25°C;
and wherein said emollient oil is selected from che group consisting of C8-C24 hydrocarbon oils, silicones, liquid diglycerides. liquid triglycerides, liquid di- and tri-glyceride derivatives, vegetable oils, liquid hydrocarbon esters, silicones, sterols, lanolins and sunscreen oils, and mixtures thereof;
the liquid hydrophilic humectant having a solubility of greater than or equal to 50% wt. in water at 25°C; said liquid humectant having a melting temperature of less than 25°C and having a viscosity at less than 5000 centipoise;
and wherein the humectant is selected from glycerol, glycerin, C1-C10 alkylene glycols such as propylene glycol, liquid polyalkylene glycols such as polypropylene glycols and polyethylene glycols with molecular weight less than 1000, ethyl hexanediol, and hexylene glycols;
(c) 15% to 70% by weight of the total composition, of a solid, amphiphilic polyol ester having the following structure described as 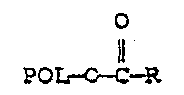 POL represents polyol moiety, R represents an organic hydrophobic group, and one or more -O-(C=O)-R functional groups are chemically attached to one or more hydroxy groups in the polyol moiety to achieve partial or total estarification;
the solid, amphiphilic polyol ester having the hydrophilic-lipophilic balance (HLB) number at between 2 and 15, said polyol ester having melting temperature at between 40°C and 90°C;
and wherein the weight ratio of the said polyol ester (c) to the sum of the said emollient oil and/or humectant (1(b)) is greater than or equal to 1:1.

2. A composition according to claim 1, wherein the surfactant or mixture of the said surfactants comprise of 10% to 60% by weight of the total composition.

3. A composition according to claims 1 or 2, wherein the surfactant or mixture of the surfactants comprise of 15% to 40% by weight of the composition.

4. A composition according to any of claims 1 to 3, wherein the hydrophobic emollient oil and/or the liquid humectant or the mixtures thereof comprise of 10% to 25% by weight of the total composition.

5. A composition according to any of claims 1 to 4, wherein the polyol ester or mixture of the said polyol esters comprise 20% to 50% by weight of the total composition.

6. A composition according to any of claims 1 to 5, wherein the polyol ester has a melting temperature of between 45°C and 70°C.

7. A composition according to any of claim 1 to 6, wherein the polyol ester has a HLB value between 2.5 and 10.

8. A composition according to claim 7, wherein the polyol ester has a HLB value between 3 and 8.

9. A composition according to any of claims 1 to 7, wherein the weight ratio of the polyol ester to the sum of the emollient oil and humectant (claim 1 (b)) is greater or equal to 1.5:1.

10. A composition according to claim 9, wherein the weight ratio of the polyol ester to the sum of the emollient oil and humectant (claim 1(b)) is greater or equal to 2:1.

11. A composition according to any of claims 1 co 10, wherein the solid, amphiphilic polyol ester (c) is selected from the group consisting of glycerin fatty esters, alkylene glycol fatty esters, pentherythritol fatty esters, polyglycerin fatty esters, and mixtures thereof.

12. A composition according to claim 11, wherein the glycerin fatty ester is glyceryl monostearate or glyceryl monolaurate.

13. A composition according to claim 11, wherein the alkylene glycol fatty ester is ethylene glycol monostearate or ethylene glycol monolaurate.

14. A composition according to claim 11, wherein the pentaeryrthrityl fatty ester is selected from pentaerythrityl monostearate and pentaerythrityl monolaurate.

15. A composition according to any of claims 1 to 14, wherein the humectant has a viscosity less than 5000 centipoise at 25°C.

16. A composition according to claim 15, wherein the hydrophobic emollient oil has a viscosity less than 1000 centipoise at 25°C.

17. A composition according to claim 16, wherein the hydrophobic emollient oil has a viscosity less than 500 centipoise at 25°C.

18. A composition according to any of claims 1 to 17, wherein the hydrophobic emollient oil has a solubility less than 5% wt. in water at 25°C;

19. A composition according to claim 18, wherein the hydrophobic emollient oil has a solubility less than 1% wt. in water at 25°C.

20. A composition according to any of claims 1 to 19, further comprising 0 to 30% by weight of the total bar composition of an optional structurant, and the total weight percentage of said optional structurant is less than the total weight percentage of said polyol ester described in (1(c)); and the optional structurant is a solid selected from C₈-C₂₄ straight and saturated fatty acid or ester derivative thereof; and/or C₈-C₂₄, straight and saturated, alcohol or ether derivatives thereof; polyalkylene glycol with molecular weight between 2000 and 20,000; starches; water soluble polymers chemically modified with hydrophobic moiety or moieties, and mixtures thereof.

21. A composition according to claim 20, wherein the optional structurant comprises 5% to 20% by weight of the total bar composition.

22. A composition according to any of claims 1 to 21, wherein the hydrophobic emollient oil is a UV absorbing sunscreening (sun protecting) oil.

23. An adjuvant chip composition comprising:
(A) 50% to 95% by weight of the chip composition of a carrier comprising a solid, amphiphilic polyol ester having the following structure: 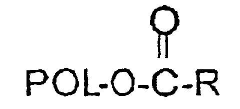 wherein POL represents polyol moiety, R represents an organic hydrophobic group, and one or more -O-(C=O)-R functional groups are chemically attached to one or more hydroxy groups in the polyol moiety to achieve partial or total esterification;
the solid, amphiphilic polyol ester having a hydrophilic-lipophilic balance (HLB) number at between 2 and 15, said polyol ester having melting temperature at between 40°C and 90°C; and
(B) 5% to 50% by weight of the chip composition of a liquid hydrophobic emollient cil or a liquid humectant or mixtures thereof;
the weight ratio of said polyol ester (A) to the sum of said emollient oil and/or humectant (B) being greater than or equal to 1:1;
the liquid hydrophobic emollient oil having a water solubility less than 10% by weight in water at 25°C; said liquid emollient oil having a melting temperature of less than 25°C; said oil having a viscosity less than 10⁵ centipoise at 25°C; and wherein said emollient oil is selected from the group consisting of C8-C24 hydrocarbon oils, silicones, liquid diglycerides, liquid triglycerides, liquid di- and tri-glyceride derivatives, vegetable oils, liquid hydrocarbon esters, silicones, sterols, lanolins and sunscreen oils, and mixtures thereof;
the liquid hydrophilic humectant having a solubility of greater than or equal to 50% wt. in water at 25°C; said liquid humectant having a melting temperature at less than 25°C and having a viscosity at less than 5000 centipoise;
and wherein said humectant is selected from polyols consisting of glycerol, glycerin, C1-C10 alkylene glycols such as propylene glycol, liquid polyalkylene glycols such as polypropylene glycols, polyethylene glycols with molecular weight less than 1000, ethyl hexanediol, and hexylene glycols;

## Patentansprüche

1. Fester Hautreinigungsriegel, umfassend:
(a) 5 % bis 70 Gew.-% auf die gesamte Riegel-Zusammensetzung Tensid oder Gemische von Tensiden; und
(b) 5 % bis 40 Gew.-% auf die gesamte Riegel-Zusammensetzung eines flüssigen hydrophoben erweichenden Öls oder eines flüssigen Feuchthaltemittels oder Gemische davon;
wobei das flüssige hydrophobe erweichende Öl bei 25°C eine Löslichkeit von weniger als 10 Gew.-% in Wasser aufweist; das flüssige erweichende Öl eine Schmelztemperatur von weniger als 25°C hat; das Öl bei 25°C eine Viskosität von weniger als 10⁵ Centipoise aufweist; und wobei das erweichende Öl ausgewählt ist aus der Gruppe, bestehend aus C₈-C₂₄-Kohlenwasserstoff-Ölen, Silikonen, flüssigen Diglyceriden, flüssigen Triglyceriden, flüssigen Di- und Triglyceridderivaten, Pflanzenölen, flüssigen Kohlenwasserstoffestern, Silikonen, Sterolen, Lanolinen und Sonnenschutzölen und Gemischen davon;
wobei das flüssige hydrophile Feuchthaltemittel bei 25°C eine Löslichkeit von größer als oder gleich 50 Gew.-% in Wasser aufweist; das flüssige Feuchthaltemittel eine Schmelztemperatur von weniger als 25°C aufweist, und eine Viskosität von weniger als 5000 Centipoise aufweist;
und worin das Feuchthaltemittel ausgewählt ist aus Glycerol, Glycerin, C₁-C₁₀-Alkylenglycolen, wie Propylenglycol, flüssigen Polyalkylenglycolen, wie Polypropylenglycolen und Polyethylenglycolen mit einem Molekulargewicht von weniger als 1000, Ethylhexandiol, und Hexylenglycolen;
(c) 15 % bis 70 Gew.-% auf die gesamte Zusammensetzung eines festen amphiphilen Polyolesters, der die nachfolgende Struktur aufweist, beschrieben als 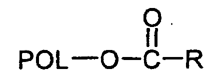 worin POL die Polyol-Einheit wiedergibt, R eine organische hydrophobe Gruppe wiedergibt, und eine oder mehrere funktionelle Gruppen -O-(C=O),-R chemisch an eine oder mehrere Hydroxygruppen in der Polyol-Einheit gebunden sind, um eine teilweise oder vollständige Veresterung zu erreichen;
wobei der feste, amphiphile Polyolester einen hydrophilen-lipophilen Ausgleichswert (HBL) zwischen 2 und 15 hat, der Polyolester eine Schmelztemperatur zwischen 40°C und 90°C aufweist;
und wobei das Gewichtsverhältnis von dem Polyolester (c) zu der Summe an dem erweichenden Öl und/oder dem Feuchthaltemittel (1(b)) größer als oder gleich 1:1 ist.

2. Zusammensetzung nach Anspruch 1, worin das Tensid oder Gemisch der Tenside 10 % bis 60 Gew.-% auf die gesamte Zusammensetzung umfasst.

3. Zusammensetzung nach Anspruch 1 oder 2, worin das Tensid oder Gemisch der Tenside 15 % bis 40 Gew.-% auf die gesamte Zusammensetzung umfasst.

4. Zusammensetzung nach einem beliebigen der Ansprüche 1 bis 3, worin das hydrophobe erweichende Öl und/oder das flüssige Feuchthaltemittel oder die Gemische davon 10 % bis 25 Gew.-% auf die gesamte Zusammensetzung umfassen.

5. Zusammensetzung nach einem beliebigen der Ansprüche 1 bis 4, worin der Polyolester oder ein Gemisch der Polyolester 20 % bis 50 Gew.-% der gesamten Zusammensetzung umfassen.

6. Zusammensetzung nach einem beliebigen der Ansprüche 1 bis 5, worin der Polyolester eine Schmelztemperatur zwischen 45°C und 70°C aufweist.

7. Zusammensetzung nach einem beliebigen von Ansprüchen 1 bis 6, worin der Polyolester einen HBL-Wert zwischen 2,5 und 10 aufweist.

8. Zusammensetzung nach Anspruch 7, worin der Polyolester einen HBL-Wert zwischen 3 und 8 aufweist.

9. Zusammensetzung nach einem beliebigen der Ansprüche 1 bis 7, worin das Gewichtsverhältnis des Polyolesters zu der Summe von erweichendem Öl und Feuchthaltemittel (Anspruch 1 (b)) größer oder gleich 1,5:1 ist.

10. Zusammensetzung nach Anspruch 9, worin das Gewichtsverhältnis des Polyolesters zu der Summe von erweichendem Öl und Feuchthaltemittel (Anspruch 1 (b)) größer oder gleich 2:1 ist.

11. Zusammensetzung nach einem beliebigen der Ansprüche 1 bis 10, worin der feste, amphiphile Polyolester (c) aus der Gruppe, bestehend aus Glycerinfettsäureestern, Alkylenglycolfettsäureestern, Pentaerythritfettsäureestern, Polyglycerinfettsäureestern und Gemischen davon, ausgewählt ist.

12. Zusammensetzung nach Anspruch 11, worin der Glycerinfettsäureester Glycerylmonostearat oder Glycerylmonolaurat ist.

13. Zusammensetzung nach Anspruch 11, worin der Alkylenglycolfettsäureester Ethylenglycolmonostearat oder Ethylenglycolmönolaurat ist.

14. Zusammensetzung nach Anspruch 11, worin der Pentaerythritylfettsäureester aus Pentaerythritylmonostearat und Pentaerythritmonolaurat ausgewählt ist.

15. Zusammensetzung nach einem beliebigen der Ansprüche 1 bis 14, worin das Feuchthaltemittel bei 25°C eine Viskosität von weniger als 5000 Centipoise aufweist.

16. Zusammensetzung nach Anspruch 15, worin das hydrophobe erweichende Öl bei 25°C eine Viskosität von weniger als 1000 Centipoise aufweist.

17. Zusammensetzung nach Anspruch 16, worin das hydrophobe erweichende Öl bei 25°C eine Viskosität von weniger als 500 Centipoise aufweist.

18. Zusammensetzung nach einem beliebigen der Ansprüche 1 bis 17, worin das hydrophobe erweichende Öl bei 25°C eine Löslichkeit von weniger als 5 Gew.-% in Wasser hat.

19. Zusammensetzung nach Anspruch 16, worin das hydrophobe erweichende Öl bei 25°C eine Löslichkeit von weniger als 1 Gew.-% in Wasser hat.

20. Zusammensetzung nach einem beliebigen der Ansprüche 1 bis 19, weiterhin umfassend 0 bis 30 Gew.-% auf die gesamte Riegel-Zusammensetzung eines wahlweisen Strukturierungsmittels, und wobei das prozentuale Gesamtgewicht des wahlweisen Strukturierungsmittels weniger als das prozentuale Gesamtgewicht des in (1(c)) beschriebenen Polyolesters liegt; und das wahlweise Strukturierungsmittel ein Feststoff ist, ausgewählt aus geradkettiger und gesättigter C₈-C₂₄-Fettsäure oder einem Esterderivat davon; und/oder aus geradkettigem und gesättigtem C₈-C₂₄-Alkohol oder Etherderivaten davon; Polyalkylenglycol mit einem Molekulargewicht zwischen 2000 und 20 000; Stärken; wasserlöslichen Polymeren, chemisch modifiziert mit einer hydrophoben Einheit oder Einheiten und Gemischen davon.

21. Zusammensetzung nach Anspruch 20, worin das wahlweise Strukturierungsmittel 5 % bis 20 Gew. -% auf die gesamte Riegel-Zusammensetzung umfasst.

22. Zusammensetzung nach einem beliebigen der Ansprüche 1 bis 21, worin das hydrophobe erweichende Öl ein UV-absorbierendes Sonnenschutzöl (vor Sonne schützendes Öl) ist.

23. Hilfsstoff-Schnitzel-Zusammensetzung, umfassend:
(A) 50 % bis 95 Gew.-% auf die Schnitzel-Zusammensetzung eines Trägers, umfassend einen festen amphiphilen Polyolester mit der nachfolgenden Struktur: 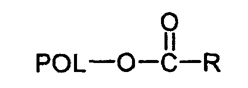 worin POL die Polyol-Einheit wiedergibt, R eine organische hydrophobe Gruppe wiedergibt, und eine oder mehrere funktionelle Gruppen -O-(C=O)-R chemisch an eine oder mehrere der Hydroxygruppen in der Polyol-Einheit gebunden sind, um eine teilweise oder vollständige Veresterung zu erreichen;
wobei der feste, amphiphile Polyolester einen hydrophilen-lipophilen Ausgleichswert (HBL) zwischen 2 und 15 aufweist, wobei der Polyolester eine Schmelztemperatur zwischen 40°C und 90°C aufweist; und
(B) 5 % bis 50 Gew.-% auf die Schnitzel-Zusammensetzung eines flüssigen erweichenden Öls oder eines flüssigen hydrophilen Feuchthaltemittels oder Gemische davon;
wobei das Gewichtsverhältnis des Polyolesters (A) zu der Summe an dem erweichenden Öl und/oder Feuchthaltemittel (B) größer als oder gleich 1:1 ist;
das flüssige hydrophobe erweichende Öl bei 25°C eine Löslichkeit in Wasser von weniger als 10 Gew.-% aufweist, das flüssige erweichende Öl eine Schmelztemperatur von weniger als 25°C aufweist, das Öl bei 25°C eine Viskosität von weniger als 10⁵ Centipoise aufweist; und worin das erweichende Öl ausgewählt ist aus der Gruppe, bestehend aus C₈-C₂₄-Kohlenwasserstoff-Ölen, Silikonen, flüssigen Diglyceriden, flüssigen Triglyceriden, flüssigen Di- und Triglyceridderivaten, Pflanzenölen, flüssigen Kohlenwasserstoffestern, Silikonen, Sterolen, Lanolinen und Sonnenschutzölen und Gemischen davon;
das flüssige hydrophile Feuchthaltemittel bei 25°C eine Löslichkeit von mehr als oder gleich 50 Gew.-% in Wasser aufweist; das flüssige Feuchthaltemittel eine Schmelztemperatur von weniger als 25°C aufweist und eine Viskosität von weniger als 5000 Centipoise aufweist;
und worin das Feuchthaltemittel aus Polyolen, bestehend aus Glycerol, Glycerin, C₁-C₁₀-Alkylenglycolen, wie Propylenglycol, flüssigen Polyalkylenglycolen, wie Polypropylenglycolen, Polyethylenglycolen mit einem Molekulargewicht von weniger als 1000, Ethylhexandiol und Hexylenglycolen, ausgewählt ist.

## Revendications

1. Pain nettoyant solide comprenant :
(a) de 5 à 70 % en poids, sur la base de la composition totale, d'un tensioactif ou d'un mélange de tensioactifs ; et
(b) de 5 % à 40 % en poids, sur la base de la composition totale du pain, d'une huile émolliente hydrophobe liquide ou d'un humectant liquide ou de mélanges de ceux-ci ;
l'huile émolliente liquide hydrophobe ayant une solubilité dans l'eau inférieure à 10 % en poids dans de l'eau à 25°C ; ladite huile émolliente liquide ayant une température de fusion inférieure à 25°C ; ladite huile ayant une viscosité inférieure à 10⁵ centipoises à 25°C ; et dans laquelle ladite huile émolliente est sélectionnée à partir du groupe constitué des huiles d'hydrocarbures en C8 - C24, des silicones, des diglycérides liquides, des triglycérides liquides, des dérivés liquides de di- et de tri - glycérides, des huiles végétales, des esters d'hydrocarbures liquides, des silicones, des stérols, des lanolines et des huiles d'écran solaire, et des mélanges de ceux-ci ;
l'humectant hydrophile liquide ayant une solubilité supérieure ou égale à 50 % en poids dans de l'eau à 25°C ; ledit humectant liquide ayant une température de fusion inférieure à 25°C et ayant une viscosité d'au moins 5.000 centipoises ;
et dans lequel l'humectant est sélectionné à partir du glycérol, de la glycérine, des alkylènes glycols en C1 - C10 tels que le propylène glycol, les polyalkylènes glycols liquides tels que les propylènes glycols et les polyéthylènes glycols ayant une masse moléculaire inférieure à 1.000, l'éthylhéxanediol et les héxylènes glycols ;
(c) de 15 % à 70 % en poids, sur la base de la composition totale, d'un ester de polyol amphiphile solide ayant la structure suivante : 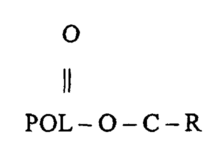 dans laquelle POL représente une partie de molécule polyol, R représente un groupe hydrophobe organique, et un ou plusieurs des groupes fonctionnels - O - (C = O) - R sont liés chimiquement à un ou plusieurs groupes hydroxy dans la partie de molécule polyol pour permettre une estérification totale ou partielle ;
l'ester de polyol amphiphile solide ayant une valeur d'équilibre hydrophile -lipophile (HLB) se situant entre 2 et 15, ledit ester de polyol ayant une température de fusion se situant entre 40°C et 90°C ;
et dans laquelle le rapport en poids entre ledit ester de polyol (c) et la somme de ladite huile émolliente et/ou de l'humectant (1(b)) est supérieure ou égale à 1 pour 1.

2. Composition selon la revendication 1, dans laquelle le tensioactif ou le mélange desdits tensioactifs constitue de 10 % à 60 % en poids de la composition totale.

3. Composition selon la revendication 1 ou 2, dans laquelle le tensioactif ou le mélange de tensioactifs constitue de 15 % à 40 % en poids de la composition totale.

4. Composition selon l'un quelconque des revendications 1 à 3, dans laquelle l'huile émolliente liquide hydrophobe et/ou l'humectant hydrophile liquide ou les mélanges de ceux-ci constituent de 10 % à 25 % en poids de la composition totale.

5. Composition selon l'une quelconque des revendications 1 à 4, dans laquelle l'ester de polyol ou le mélange desdits esters de polyol constitue de 20 % à 50 % en poids de la composition totale.

6. Composition selon l'une quelconque des revendications 1 à 5, dans laquelle l'ester de polyol a un point de fusion se situant entre 45°C et 70°C.

7. Composition selon l'une quelconque des revendications 1 à 6, dans laquelle l'ester de polyol a une valeur HLB se situant entre 2,5 et 10.

8. Composition selon la revendication 7, dans laquelle l'ester de polyol a une valeur HLB se situant entre 3 et 8.

9. Composition selon l'une quelconque des revendications 1 à 7, dans laquelle le rapport en poids entre l'ester de polyol et la somme d'huile émolliente et d'humectant (revendication 1(b)) est supérieur ou égal à 1,5 pour 1.

10. Composition selon la revendication 9, dans laquelle le rapport en poids entre l'ester de polyol et la somme d'huile émolliente et d'humectant (revendication 1(b)) est supérieur ou égal à 2 pour 1.

11. Composition selon l'une quelconque des revendications 1 à 10, dans laquelle l'ester de polyol amphiphile solide (c) est sélectionné à partir du groupe constitué des esters gras de glycérine, des esters gras d'alkylène, des esters gras de pentaérythritol, des esters gras de polyglycérine et des mélanges de ceux-ci.

12. Composition selon la revendication 11, dans laquelle l'ester gras de glycérine est du monostéarate de glycéryle ou du monolaurate de glycéryle.

13. Composition selon la revendication 11, dans laquelle l'ester gras d'alkylène glycol est du monostéarate de glycol ou du monolaurate d'éthylène glycol.

14. Composition selon la revendication 11, dans laquelle l'ester gras de pentaérythrityle est sélectionné à partir du monostéarate de pentaérythrityle et du monolaurate de pentaérythrityle.

15. Composition selon l'une quelconque des revendications 1 à 14, dans laquelle l'humectant a une viscosité inférieure à 5.000 centipoises à 25°C.

16. Composition selon la revendication 15, dans laquelle l'huile émolliente a une viscosité inférieure à 1.000 centipoises à 25°C.

17. Composition selon la revendication 16, dans laquelle l'huile émolliente hydrophobe a une viscosité inférieure à 500 centipoises à 25°C.

18. Composition selon l'une quelconque des revendications 1 à 17, dans laquelle l'huile émolliente hydrophobe a une solubilité inférieure à 5 % en poids dans de l'eau à 25°C

19. Composition selon la revendication 16, dans laquelle l'huile émolliente hydrophobe a une solubilité inférieure à 1 % en poids dans de l'eau à 25°C.

20. Composition selon l'une quelconque des revendications 1 à 19, comprenant en outre de 0 à 30 % en poids, sur la base de la composition totale du pain, d'un structurant optionnel, et le pourcentage en poids total dudit structurant optionnel est inférieur au pourcentage en poids total dudit ester de polyol décrit en (1(c)) ; le structurant optionnel étant un solide sélectionné à partir de l'acide gras en C8 - C24 à chaîne droite et saturée ou d'un dérivé ester de celui-ci ; et/ou d'un alcool en C8 - C24 à chaîne droite et saturée ou des dérivés éthers de celui-ci ; du polyalkylène glycol ayant une masse moléculaire comprise entre 2.000 et 20.000 ; des amidons ; des polymères solubles dans l'eau et modifiés chimiquement avec une ou plusieurs parties de molécules hydrophobes, et des mélanges de ceux-ci.

21. Composition selon la revendication 20,dans laquelle le structurant optionnel constitue de 5 % à 20 % en poids de la composition totale du pain.

22. Composition selon l'une quelconque des revendications 1 à 21, dans laquelle l'huile émolliente hydrophobe est une huile d'écran solaire (de protection contre le soleil) qui absorbe les UV.

23. Composition de copeaux adjuvants comprenant :
(A) de 50 % à 95 % en poids, sur la base de la composition des copeaux, d'un matériau formant support comprenant un ester de polyol amphiphile solide ayant la structure suivante : 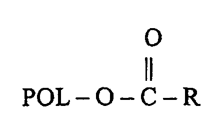 dans laquelle POL représente une partie de molécule hydrophobe, R représente un groupe hydrophobe organique, et un ou plusieurs groupes fonctionnels - O - (C = O) - R sont chimiquement fixés à un ou plusieurs groupes hydroxy dans la partie de molécule polyol afin de permettre une, estérification totale ou partielle ;
l'ester de polyol amphiphile solide ayant une valeur d'équilibre hydrophile - lipophile (HLB) se situant entre 2 et 15, ledit ester de polyol ayant une température de fusion se situant entre 40°C et 90°C; et
(B) de 5 % à 50 % en poids, sur la base de la composition de copeaux, d'une huile émolliente liquide hydrophobe ou d'un humectant liquide ou de mélanges de ceux-ci ;
le rapport en poids entre ledit ester de polyol (A) et la somme de ladite huile émolliente et/ou dudit humectant (B) étant supérieur ou égal à 1 pour 1 ;
l'huile émolliente hydrophobe liquide ayant une solubilité inférieure à 10 % en poids dans de l'eau à 25°C ; ladite huile liquide émolliente ayant une température de fusion inférieure à 25°C ; ladite huile d'olive ayant une viscosité inférieure à 10⁵ centipoises à 25°C ; et dans laquelle ladite huile émolliente est sélectionnée à partir du groupe constitué des huiles hydrocarbures en C₈ - C₂₄, des silicones, des diglycérides liquides, des dérivés liquides de di- et de tri-glycérides, des huiles végétales, des esters d'hydrocarbures liquides, des silicones, des stérols, des lanolines et des huiles d'écran solaire, et des mélanges de ceux-ci ;
l'humectant hydrophile liquide ayant une solubilité supérieure ou égale à 50 % en poids dans de l'eau à 25°C ; ledit humectant liquide ayant une température de fusion inférieure à 25°C et ayant une viscosité inférieure à 5.000 centipoises ;
et dans laquelle ledit humectant est sélectionné à partir des polyols constitués du glycérol, de la glycérine, des alkylènes glycols en C₁- C₁₀ tels que le propylène glycol, des polyalkylènes glycols liquides tels que les propylènes glycols, des polyéthylènes glycols ayant une masse moléculaire inférieure à 1.000, de l'éthyle héxanediol et des héxylènes glycols.
